# EUROPÄISCHE PATENTSCHRIFT

(11) **EP 0 863 401 B1**
(45) Veröffentlichungstag und Bekanntmachung des Hinweises auf die Patenterteilung: **28.08.2002**
(21) Anmeldenummer: 98103650.2
(22) Anmeldetag: 03.03.1998
(51) Int. Cl.: G01N 33/52, G01N 33/94, G01N 33/535, G01N 33/566, C12Q 1/42

(54) **Analytisches Testelement mit flüssigkeitsgefüllten Blister**
Analytical test device with liquid-filled blister
Elément d'essai analytique avec blister à remplissage liquide

(30) Priorität: 06.03.1997 DE 19709090
(43) Veröffentlichungstag der Anmeldung: 09.09.1998
(73) Patentinhaber: Roche Diagnostics GmbH, 68298 Mannheim (DE)
(72) Erfinder: Harttig, Herbert, Dr., 67122 Altrip (DE); Klein, Christian, Dr., 82362 Weilheim (DE)

(56) Entgegenhaltungen:
- EP-A- 0 279 574

## Beschreibung

Die Erfindung betrifft ein analytisches Testelement enthaltend auf einem Träger saugfähiges Material mit Probenauftrag- und Nachweiszone, sowie einen flüssigkeitsgefüllten Blister. Außerdem betrifft die Erfindung ein Verfahren zur Herstellung eines solchen analytischen Testelementes und ein Verfahren zur Bestimmung eines Analyts mit einem solchen analytischen Testelement.

Zur qualitativen oder quantitativen analytischen Bestimmung von Probenbestandteilen werden oft sogenannte trägergebundene Tests verwendet. Bei diesen sind Reagenzien in entsprechenden Schichten eines analytischen Testelementes eingebettet, das mit der Probe in Kontakt gebracht wird. In der Regel laufen die Nachweisreaktionen in flüssiger Phase ab. Diese Flüssigkeit stammt bei flüssigen Proben meist von der Probe selbst oder die Flüssigkeit wird zu der Probe auf das analytische Testelement aufgegeben. Die Reaktion von Probe und Reagenzien führt dann zu einem nachweisbaren Signal, insbesondere einem Farbumschlag, welcher visuell oder mit Hilfe eines Gerätes, meistens reflektionsphotometrisch, ausgewertet werden kann. Elektrochemische Nachweisverfahren sind mittlerweile ebenfalls auf analytischen Testelementen möglich.

Analytische Testelemente sind häufig als Teststreifen ausgebildet, die im wesentlichen aus einem länglichen Träger, das heißt, einer Tragschicht, beispielsweise aus Kunststoffmaterial, und darauf angebrachten Nachweisschichten als Testfelder bestehen. Es sind jedoch auch analytische Testelemente bekannt, die als quadratische oder rechteckige Plättchen gestaltet sind.

Analytische Testelemente der eingangs bezeichneten Art sind beispielsweise aus der europäischen Patentschrift 0 279 574 bekannt. Das dort beschriebene analytische Testelement enthält innerhalb eines flüssigkeitsundurchlässigen Gehäuses eine saugfähige Schicht mit einer Probenauftrag- und einer Nachweiszone sowie einen flüssigkeitsgefüllten Blister aus einem zerreißbaren, besonders geformten Material. Der Blister ist so aus steifem aber dennoch deformierbarem Plastik geformt, daß er eine gewölbte Oberfläche besitzt, in der eine oder mehrere nach innen gerichtete spitze, feste Einstülpungen ausgebildet sind. Bei Druck von außen auf diese spitzen Einstülpungen wird die gegenüberliegende Blisterwand durchstochen und die im Blister befindliche Flüssigkeit tritt aus. Im Kontakt mit der saugfähigen Schicht wird die Flüssigkeit von dieser Schicht aufgenommen und breitet sich dort durch Kapillarkraft aus. In der Flüssigkeit wird dann das zu untersuchende Probenmaterial mit Nachweisreagenzien zur Reaktion gebracht. Die besondere Form des Blisters ist nur relativ aufwendig herzustellen. Außerdem ist die gesamte Herstellung solcher mit Flüssigkeit gefüllter Blister nur diskontinuierlich möglich, weil erst die Formgebung mittels Spritzguß oder Tiefziehverfahren erfolgen muß, anschließend mit Flüssigkeit gefüllt und abschließend mit einer durchstoßbaren Folie flüssigkeitsdicht versiegelt werden muß.

Die Nachteile des Standes der Technik werden durch die Erfindung beseitigt, wie sie in den Patentansprüchen näher charakterisiert ist.

Demgemäß ist Gegenstand der Erfindung ein analytisches Testelement, das auf einem Träger saugfähiges Material mit Probenauftrag- und Nachweiszone sowie einen flüssigkeitsgefüllten Blister enthält, wobei der Träger einen Einschnitt in der Trägerfläche aufweist. Dieser Einschnitt ist so geformt, daß beim Biegen des Trägers dort eine Spitze entsteht, mit der der Blister so geöffnet werden kann, daß Flüssigkeit austritt und in Kontakt mit dem saugfähigen Material gelangt.

Gegenstand der Erfindung ist außerdem ein Verfahren zur Herstellung eines solchen analytischen Testelementes. Hierzu wird auf eine Schicht aus biegbarem aber dennoch steifem Material, das nebeneinander Einschnitte aufweist, saugfähiges Material und ein kontinuierliches Band flüssigkeitsgefüllter Blister aufgebracht. In dem kontinuierlichen Band sind die einzelnen Blister durch Querschweißnähte getrennt. Das Band wird so auf die Schicht aus biegbarem aber dennoch steifem Material aufgebracht, daß jeweils ein zwischen zwei Querschweißnähte befindlicher Blister über einem Einschnitt innerhalb der Trägerfläche zu liegen kommt. Abschließend entstehen durch Zerschneiden der biegbaren aber dennoch steifen Schicht entlang der Querschweißnähte im Blisterband einzelne analytische Testelemente.

Schließlich ist Gegenstand der Erfindung auch ein Verfahren zur Bestimmung eines Analyts mittels eines erfindungsgemäßen analytischen Testelementes. Hierzu wird eine Probe in Kontakt mit der Probenauftragzone gebracht, die Trägerschicht gebogen und mit der entstandenen Spitze der flüssigkeitsgefüllte Blister geöffnet. Ausgetretene Flüssigkeit kontaktiert die Probe. Der Nachweis des zu bestimmenden Analyts erfolgt dann entweder in der Probenauftragzone selbst oder in einer Nachweiszone, in die die Flüssigkeit transportiert wird.

Ein wesentlicher Bestandteil des erfindungsgemäßen analytischen Testelementes ist der Träger. Er muß einerseits so steif sein, daß er die Funktionselemente, im wesentlichen das saugfähige Material mit Probenauftrag- und Nachweiszone, sowie den flüssigkeitsgefüllten Blister tragen und der Anwender das analytische Testelement handhaben kann. Andererseits muß der Träger so gebogen werden können, daß dabei aus dem Einschnitt eine Spitze entsteht, die ihrerseits wieder so steif und fest sein muß, daß der flüssigkeitsgefüllte Blister geöffnet werden kann. Hierfür kommen insbesondere Plastikmaterialien in Frage, die an der Einschnittstelle so gebogen werden können, daß die notwendige Spitze freigelegt wird. Entsprechende Polyester-, Polystyrol- oder Polyamidfolien haben sich hierfür als vorteilhaft erwiesen. Um das Biegen des Trägers an der gewünschten Stelle zu erleichtern, kann dort auch eine Sollbiegestelle vorgesehen sein. Diese kann beispielsweise aus einem entsprechend dünnerem Materialquerschnitt bestehen oder es können seitliche Einkerbungen angebracht sein, die das Biegen des Trägers an vorbestimmter Stelle erleichtern.

Der Einschnitt in der Trägerfläche ist vorzugsweise V-förmig, so daß beim Biegen des Trägers entlang einer Linie durch die oberen Schenkel des V eine Spitze entsteht. Es sind aber auch andere Einschnitte möglich, so lange damit beim Biegen des Trägers spitze Formen entstehen, mit der der flüssigkeitsgefüllte Blister geöffnet werden kann. Beispielsweise sind auch W-förmige Einschnitte möglich, durch die beim Biegen des Trägers zwei Spitzen entstehen, mit denen der Blister geöffnet werden kann.

Über der Einschnittstelle in der Trägerfläche muß ein flüssigkeitsgefüllter Blister so angeordnet sein, daß er beim Biegen des Trägers durch die freigelegte Spitze(n) des Einschnitts geöffnet werden kann. Hierzu kann der Blister direkt über dem Einschnitt auf der Trägerfläche liegen und das saugfähige Material mit Probenauftrag- und Nachweiszone kann daneben angeordnet sein, so daß beim Öffnen des Blisters austretende Flüssigkeit durch das saugfähige Material aufgenommen wird oder der Blister befindet sich über der Einschnittstelle des Trägers auf zwischen Blister und Träger angeordnetem saugfähigen Material, so daß die beim Biegen des Trägers freigelegte Spitze durch das saugfähige Material hindurch mit dem Blister in Kontakt kommt und diesen öffnet. Das saugfähige Material kann in letzterem Fall an der Stelle des Einschnitts in der Trägerfläche eine Aussparung aufweisen oder es kann auch ohne eine solche Aussparung auf dem Träger angeordnet sein. Falls keine Aussparung in dem saugfähigen Material vorhanden ist, muß es so beschaffen sein, daß es beim Biegen des Trägers von der freigelegten Spitze durchstoßen werden kann, so daß die Spitze auch den auf dem saugfähigen Material befindlichen Blister öffnen kann.

Als saugfähige Materialien kommen grundsätzlich alle solche in Frage, die generell in sogenannten "Trockentesten" zur Flüssigkeitsaufnahme eingesetzt werden können. Als vorteilhaft haben sich hierfür beispielsweise Membranen erwiesen. Ganz besonders bevorzugt sind jedoch faserige, saugfähige Matrixmaterialien wie Vliese, Gewebe oder Gewirke. Vliese sind ganz besonders bevorzugt. Die fasrigen Matrixmaterialien können Glas-, Zellulose-, Polyesterfasern aber auch Viskose und Polyvinylalkohol enthalten. Vliesmaterialien, enthaltend schmelzbare Copolyesterfasern neben Glasfasern, Polyesterfasern, Polyamidfasern, Zellulosefasern oder Zellulosederivatefasern, wie sie in der europäischen Patentanmeldung 0 571 941 beschrieben sind, können ebenfalls vorteilhaft in dem erfindungsgemäßen analytischen Testelement eingesetzt werden. Papiere, wie beispielsweise Teebeutelpapier, sind ebenfalls gut einsetzbar.

Als Blister können grundsätzlich alle flüssigkeitsgefüllten Elemente verwendet werden, die mit einem spitzen Gegenstand, wie er beim Biegen des Trägers des erfindungsgemäßen analytischen Testelementes entsteht, geöffnet werden können. Vorteilhaft hierfür haben sich solche Elemente erwiesen, die die Flüssigkeit mit einer dünnen Haut aus Metall, Kunststoff oder kunststoffbeschichtetem Metall einschließen. Erfindungsgemäß besonders bevorzugt sind solche Blister, die aus einer kunststoffbeschichteten, dünnen Metallfolie, vorzugsweise aus Aluminium, hergestellt sind.

Zur Herstellung solcher besonders bevorzugter Blister werden beispielsweise die seitlichen Ränder einer kunststoffkaschierten Metallfolie thermisch miteinander verschweißt, so daß sich ein kunststoffkaschierter Metallschlauch bildet. Durch eine thermische Querverschweißung, das heißt, thermische Siegelung quer zur Längsrichtung des Schlauches, wird ein nach unten verschlossenes Behältnis geschaffen, in das von oben durch die Schlauchöffnung Flüssigkeit eingefüllt werden kann. Durch erneute Querverschweißung oberhalb des Flüssigkeitsniveaus oder durch die Flüssigkeit hindurch und mehrmalige Wiederholung dieses Vorganges wird ein flüssigkeitsgefülltes Band erhalten, das eine Vielzahl von Blistern aneinandergereiht enthält. Wenn gewünscht, können diese Blister anschließend oder zu einem späteren Zeitpunkt durch Zerschneiden des Bandes entlang der Querschweißnähte vereinzelt werden.

Damit beim Biegen des Trägers der flüssigkeitsgefüllte Blister nicht dem Druck der freigelegten Spitze ausweichen kann, muß er entweder manuell, beispielsweise mit dem Finger, oder durch konstruktive Maßnahmen festgehalten werden. Dazu kann der Blister zum Beispiel entlang seiner Querschweißnähte, parallel der Längsrichtung des Testelementes und damit längs des Flüssigkeitstransportes innerhalb des Testelementes nach Öffnen des Blisters auf dem Träger befestigt werden. Eine solche Befestigung ist beispielsweise mittels doppelseitigem Klebeband möglich. Vorzugsweise erfolgt diese Befestigung mit Schmelzkleberstreifen. In einer ganz besonders bevorzugten Ausführungsform wird der Blister nicht nur auf den beiden Längsseiten des Testelements befestigt, sondern auch an der der Flüssigkeitstransportrichtung entgegengesetzten Seite des Testelementes. Beim Öffnen des Blisters wird so besonders vorteilhaft austretende Flüssigkeit von dem saugfähigen Material, das dem Blister benachbart ist, aufgenommen.

Eine andere, ebenfalls effektive Montagemethode des Blisters auf dern analytischen Testelement beinhaltet die Applikation einer Folie, welche den Blister überdeckt und sich beim Biegen des Trägers fest über den flüssigkeitsgefüllten Blister spannt, so daß dieser nicht ausweichen kann. Eine solche Folie ist auf den Längsseiten des Trägers befestigt, damit der Flüssigkeitstransport im Testelement nicht gestört wird. Bei einer solchen Ausfühungsform kann unter Umständen darauf verzichtet werden, den Blister direkt auf dem Träger zu befestigen. Eine solche Folie hat auch eine mechanische Schutzfunktion, durch die verhindert wird, daß der Blister unbeabsichtigt durch spitze Gegenstände geöffnet wird. Darüberhinaus kann eine solche Folie bei entsprechender Zusammensetzung auch als Lichtschutz für die im Blister befindliche Flüssigkeit dienen.

Eine weitere wirksame Methode zum Festhalten des Blisters beim Biegen des Trägers des erfindungsgemäßen Testelementes besteht im Einbau des Testelementes in ein stabiles Gehäuse, welches den Blister ausreichend weit überdeckt, um einerseits ein Ausweichen vor der beim Biegen entstehenden Spitze zu verhindern, aber andererseits ein Biegen des Trägers des Testelementes noch erlaubt.

Die in dem Blister des erfindungsgemäßen analytischen Testelementes befindliche Flüssigkeit kann eine Elutionsflüssigkeit sein, die dazu dient, Analyt und Reagenzien, die sich auf dem saugfähigen Material des analytischen Testelementes befinden, mit ausreichend Flüssigkeit zum Ermöglichen einer Reaktion in Kontakt zu bringen und / oder den Transport von Probenmaterial und / oder Reagenzien im saugfähigen Material durch Chromatographie zu ermöglichen. Die Blisterflüssigkeit kann aber auch alle oder Teile der benötigen Reagenzien enthalten, die für den Nachweis des zu bestimmenden Analyt auf dem erfindungsgemäßen analytischen Testelement notwendig sind.

Dementsprechend kann das saugfähige Material unterschiedlich aufgebaut sein. Auf jeden Fall muß das saugfähige Material so in Relation zu dem flüssigkeitsgefüllten Blister angeordnet sein, daß aus dem Blister austretende Flüssigkeit von dem saugfähigen Material aufgenommen wird. Das saugfähige Material des erfindungsgemäßen analytischen Testelements enthält mindestens eine Probenauftrag- und eine Nachweiszone. Im einfachsten Falle sind Probenaufgabeund Nachweiszone bezüglich des saugfähigen Materials identisch. Die entsprechende Zone ist so in Relation zu dem flüssigkeitsgefüllten Blister angeordnet, daß nach der Öffnung des Blisters Flüssigkeit, die aus dem Blister austritt von dem saugfähigen Material der Probenaufgabe- und Nachweiszone aufgenommen wird. Im Falle der Identität von Probenaufgabeund Nachweiszone wird die Blisterflüssigkeit vorteilhafterweise zumindest einen Teil der für die Nachweisreaktion erforderlichen Reagenzien enthalten.

Es ist aber auch möglich, daß sich Probenaufgabe- und Nachweiszone in unterschiedlichen Bereichen des saugfähigen Materials befinden. In diesem Fall kann das saugfähige Material auch noch einen oder mehrere zusätzliche Bereiche aufweisen, die Reagenzien enthalten, die für den Nachweis des zu bestimmenden Analyt erforderlich sind. Die verschiedenen Zonen können auf dem gleichen saugfähigen Material enthalten sein. Sie können aber auch auf verschiedenen saugfähigen Materialien vorliegen. Im Grunde genommen sind alle möglichen Aufbauten für analytische Testelemente möglich, so lange die Probenaufgabezone bzw. Elutionsmittelauftragezone so in Verbindung mit dem flüssigkeitsgefüllten Blister steht, daß beim Öffnen des Blisters Flüssigkeit in die Probenaufgabezone gelangt und entweder dort, bei Identität von Probenaufgabe- und Nachweiszone eine Detektion des zu bestimmenden Analyts ermöglicht oder von dort Probenmaterial in die Nachweiszone, gegebenenfalls über dazwischen geschaltete weitere Reagenzien enthaltende Zonen transportiert wird.

Das erfindungsgemäße analytische Testelement ist einfach herstellbar. Wie bereits zuvor beschrieben, wird ein flüssigkeitsgefülltes Blisterband hergestellt, das auf ein Trägerband, das nebeneinander angeordnet Einschnitte innerhalb der Trägerfläche besitzt, die beim Biegen des Trägers ein Spitze entstehen lassen, und das bereits das saugfähige Material trägt, so aufgebracht, daß ein Blister zwischen zwei Querschweißnähten jeweils über einen Einschnitt innerhalb der Trägerfläche zu liegen kommt. Außerdem ist es wichtig, daß das saugfähige Material so in Relation zu dem Blisterband angeordnet ist, daß bei Flüssigkeitsaustritt aus dem Blister diese von dem saugfähigen Material aufgenommen wird. Anschließend können durch Schneiden des Trägerbandes entlang der Querschweißnähte im Blisterband die analytischen Testelemente gemäß der vorliegenden Erfindung vereinzelt werden.

Das beschriebene analytische Testelement ist besonders einfach handhabbar für analytische Untersuchungen, die Flüssigkeit erfordern. Erfindungsgemäß ist es ausreichend, wenn zu untersuchendes Probenmaterial in Kontakt mit der Probenauftragszone gebracht wird, der Träger gebogen und mit der so entstandenen Spitze in der Trägerfläche der flüssigkeitsgefüllte Blister geöffnet wird. Aus dem Blister ausgetretene Flüssigkeit kontaktiert die Probe und führt, je nach vorstehend beschriebenem Aufbau des erfindungsgemäßen analytischen Testelementes, in der Probenaufgabezone selbst oder in einer hiervon separaten Nachweiszone, in die der zu untersuchende Analyt mit der Flüssigkeit transportiert wird, zur Detektion der zu bestimmenden Substanz. Die zu untersuchende Probe kann fest oder flüssig sein. Sie kann auf das analytische Testelement aufgetragen werden oder das analytische Testelement kann mit der zu untersuchenden Probe kontaktiert werden. So ist es möglich, daß flüssige Probe auf die Probenaufgabezone des analytischen Testelementes aufgegeben wird. Es ist aber auch möglich, daß das analytische Testelement mit der Probenaufgabezone in die zu untersuchende flüssige Probe eingetaucht wird, bis die Probenaufgabezone mit zu untersuchender Flüssigkeit gefüllt ist und dann wieder aus der Flüssigkeit herausgezogen wird. Feste Substanzen können ebenfalls auf die Probenaufgabezone aufgegeben werden. Es ist aber auch möglich, daß beispielsweise mit der Probenaufgabezone über feste Oberflächen gewischt wird und so feste oder flüssige Substanzen auf die Probenaufgabezone gelangen, die anschließend untersucht werden.

Auch gasförmige Substanzen können mit dem erfndungsgemäßen analytischen Testelement untersucht werden. Hierfür kann beispielsweise Probenaufgabe- und Nachweiszone identisch sein. Reagenz zum Nachweis des gasförmigen Analyt enthaltende Flüssigkeit im Blister kann durch Biegen des analytischen Testelementes freigesetzt werden und wird vom saugfähigen Material absorbiert. Gasförmiger Analyt, der mit der feuchten, Reagenz enthaltenden Nachweiszone in Kontakt kommt, wird dort zu einem detektierbaren Signal führen.

Als detektierbares Signal kommt bei allen erfindungsgemäßen Analyseverfahren insbesondere ein Farbumschlag in Frage, welcher visuell oder mit Hilfe eines Gerätes, meistens reflektionsphotometrisch ausgewertet werden kann. Farbumschlag bedeutet hierbei eine Farbveränderung, wobei damit auch das Entstehen einer Farbe (der Übergang farblos zu farbig) oder das Verschwinden einer Farbe (Übergang farbig zu farblos) verstanden wird. Auch die Intensitätsänderung einer Farbe soll unter dem Begriff "Farbumschlag" verstanden werden. Darüberhinaus kann ein detektierbares Signal auch eine Fluoreszenzbildung oder -löschung sein. Ebenso können auch elektrochemische Signale für die Bestimmung von Analyten genutzt werden, wie Stromstärke oder Spannung, wenn das Testelement mit entsprechenden Elektroden in der Nachweiszone ausgestattet ist.

In den Fig. 1-7 sind besonders vorteilhafte erfindungsgemäße analytische Testelemente, ein Verfahren zur Herstellung flüssigkeitsgefüllter Blister für solche Testelemente und wichtige Stadien des Herstellungsverfahrens erfindungsgemäßer analytischer Testelemente dargestellt.

Fig. 1 A bis C zeigen ein erfindungsgemäßes analytisches Testelement in Aufsicht von unten, Aufsicht von oben und in Seitenansicht. Fig. 1 D zeigt die Seitenansicht eines erfindungsgemäßen analytischen Testelementes im gebogenen Zustand.

Fig. 2 A bis C zeigen die Aufsicht von unten, Aufsicht von oben und Seiteneinsicht eines weiteren besonders bevorzugten erfindungsgemäßen analytischen Testelementes.

Fig. 3 und 4 zeigen Stadien der Herstellung eines flüssigkeitsgefüllten Blisterbandes.

Fig. 5 -7 zeigen wesentliche Stadien bei der Herstellung eines erfindungsgemäßen analytischen Testelementes nach Fig. 1.

Fig. 8 zeigt die Seitenansicht eines weiteren besonders bevorzugten erfindungsgemäßen analytischen Testelementes.

### Bezugszeichenliste

- 1 =: Träger
- 2 =: Einschnitt
- 3 =: Spitze
- 4 =: saugfähiges Material
- 5 =: Probenauftragzone
- 6 =: Nachweiszone
- 7 =: Blister
- 8 =: Schmelzkleber
- 9 =: Reagenzzone
- 10 =: Kunststoffbeschichtete Metallfolie
- 11 =: Siegelrollen
- 12 =: Längsschweißnaht
- 13 =: Kunststoffbeschichteter Metallschlauch
- 14 =: Heizbacken
- 15 =: Querschweißnaht
- 16 =: Blisterflüssigkeit
- 17 =: Blisterband
- 18 =: Positionierloch
- 19 =: Blister tragende Zone

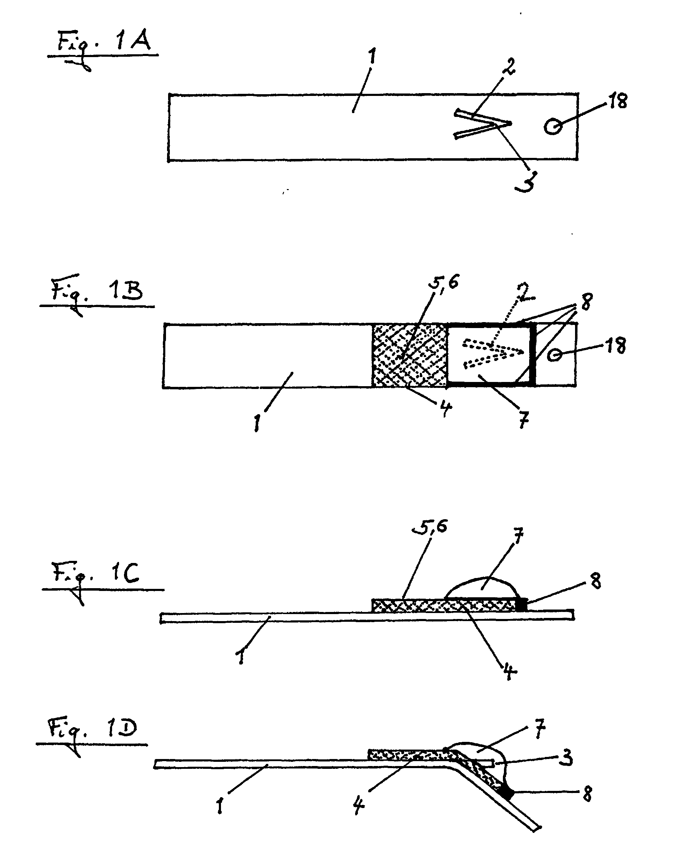

Ein bevorzugtes erfindungsgemäßes analytisches Testelement ist in Fig. 1 gezeigt. Wie von der Unterseite des Testelementes, wie es in Fig. 1 A dargestellt ist, erkennbar, befindet sich in der Fläche des Trägers (1) ein Einschnitt (2), der beim Biegen des Trägers (1) eine Spitze (3) entstehen läßt. Im vorliegenden Fall handelt es sich um einen V-förmigen Einschnitt (2). Wie bereits zuvor ausgeführt, können aber auch andere Formen von Einschnitten zur Anwendung kommen. Wichtig ist nur, daß beim Biegen des Trägers (1) eine Spitze (3) freigelegt wird, mit der der flüssigkeitsgefüllte Blister, der sich auf dem analytischen Testelement befindet, geöffnet werden kann. In dem gezeigten, bevorzugten analytischen Testelement befindet sich auch ein Positionierloch (18) in dem Träger (1), das die positionsgenaue Montage der Einzelbestandteile des analytischen Testelementes bei dessen Herstellungsprozess ermöglicht.

Auf der Oberseite des Trägers (1), die in Fig. 1 B dargestellt ist, ist das saugfähige Material (4) angeordnet. Am Rand des saugfähigen Materials (4) und des Trägers (1) ist der flüssigkeitsgefüllte Blister (7) so mit Schmelzkleberstreifen (8) befestigt, daß er sich über dem Einschnitt (2) des Trägers (1) befindet. Das saugfähige Material ist größer als die Grundfläche des Blisters (7), so daß neben dem Blister (2) eine Fläche des saugfähigen Materials (4) unbedeckt durch den Blister (7) freiliegt. Diese Fläche des saugfähigen Materials (4) enthält die Probenauftragzone (5) und die Nachweiszone (6) des erfindungsgemäßen analytischen Testelementes. Im Fall des bevorzugten analytischen Testelementes, das in Fig. 1 gezeigt ist, sind Probenauftragzone (5) und Nachweiszone (6) identisch, das heißt, die zu untersuchende Probe wird auf den Bereich des saugfähigen Materials (4) aufgegeben, in dem auch der Nachweis des zu bestimmenden Analyts erfolgt. Der Blister (7) darfnicht so befestigt sein, daß beim Flüssigkeitsaustritt Aufnahme der Flüssigkeit in das saugfähige Material (4) und Transport der Flüssigkeit in die Probenaufgabezone (5) und Nachweiszone (6) verhindert oder in störendem Ausmaß beeinflußt wird. Im vorliegenden Beispiel ist der Blister (7) mit Schmelzkleberstreifen (8) auf drei Seiten befestigt, so daß beim Biegen des Trägers (1) der Blister (7) der freigelegten Spitze (3) des Einschnitts (2) nicht ausweichen kann.

Zur Durchführung einer Analytbestimmung wird auf das in Fig. 1 C im Querschnitt gezeigte bevorzugte analytische Testelement das zu untersuchende Probenmaterial auf die Probenauftragzone (5) des saugfähigen Materials (4) aufgebracht. Beim Biegen des Trägers (1), wie es im Querschnitt in Fig. 1 D gezeigt ist, dringt die Spitze (3) in den flüssigkeitsgefüllten Blister (7) ein und Flüssigkeit tritt aus dem Blister (7) aus. Die Flüssigkeit wird von dem saugfähigen Material (4) aufgenommen und breitet sich dort in Richtung der Probenauftragszone (5) aus. In dem dann dort vorliegenden flüssigen Medium findet eine Nachweisreaktion statt, die zu einem Signal führt, das in der mit der Probenaufgabezone (5) identischen Nachweiszone (6) beobachtet werden kann. In einer bevorzugten Ausführungsform wird es sich um ein Farbsignal handeln, das entweder von oben oder von unten durch den Träger (1) beobachtet werden kann, wenn der Träger (1) entsprechend beschaffen ist. Für eine Beobachtung durch den Träger (1) muß der Träger (1) im Bereich der Nachweiszone (6) entweder transparent sein oder eine Öffnung aufweisen, durch die das saugfähige Material (4) im Bereich der Nachweiszone (6) beobachtbar ist. Die für die Nachweisreaktion erforderlichen Reagenzien können sich entweder vollständig in oder auf dem saugfähigen Material (4) befinden oder sie können in der Flüssigkeit des Blisters (7) enthalten sein. Es ist natürlich auch möglich, daß Teile des Nachweisreagenzes sich in der Flüssigkeit des Blisters (7) befinden, andere Teile in oder auf dem saugfähigen Material (4). Eine Aufteilung des Nachweisreagenzes ist vor allem dann notwendig, wenn es sich um solche Reagenzbestandteile handelt, die bei direktem Kontakt miteinander eine störende Wechselwirkung eingehen würden.

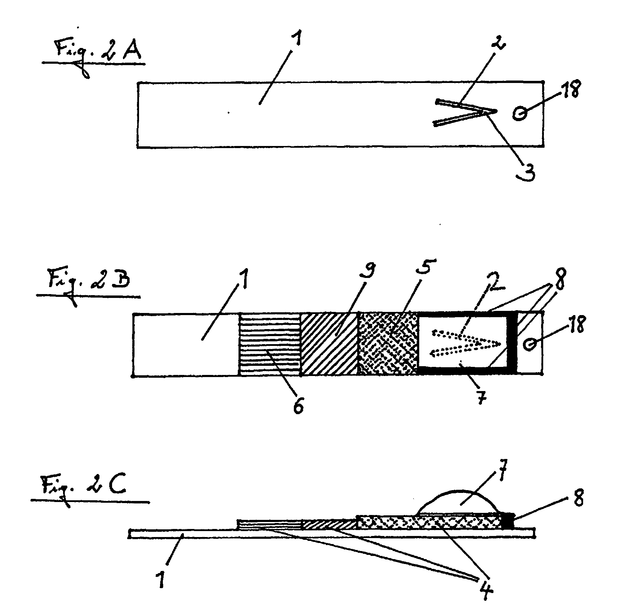

In Fig. 2 ist eine andere bevorzugte Ausführungsform eines analytischen Testelementes dargestellt. Hierbei ist das saugfähige Material (4) in unterschiedliche, nebeneinander angeordnete Funktionsbereiche aufgeteilt. Dem Blister (7) direkt benachbart ist die Probenauftragzone (5), an die sich eine Reagenzzone (9) anschließt, in der sich für die Nachweisreaktion erforderliche Reagenzien befinden. Die Bestimmung des Analyts erfolgt in der Nachweiszone (6), die neben der Reagenzzone (9) angeordnet ist, so daß sich die Reagenzzone (9) zwischen Probenauftragzone (5) und Nachweiszone (6) angeordnet befindet. Ein solches erfindungsgemäßes analytisches Testelement ist besonders dann vorteilhaft einsetzbar, wenn nach der Probenaufgabe noch einige Zeit mit dem Start und der Durchführung der Nachweisreaktion gewartet werden soll. Wenn sich in der Probenauftragzone probenstabilisierende Substanzen befinden, kann die Probe nach Aufbringen auf die Probenauftragzone dort längere Zeit aufbewahrt werden, bevor die Nachweisreaktion durch Biegen des Trägers (1) und Öffnen des flüssigkeitsgefüllten Blisters (7) gestartet und durchgeführt wird.

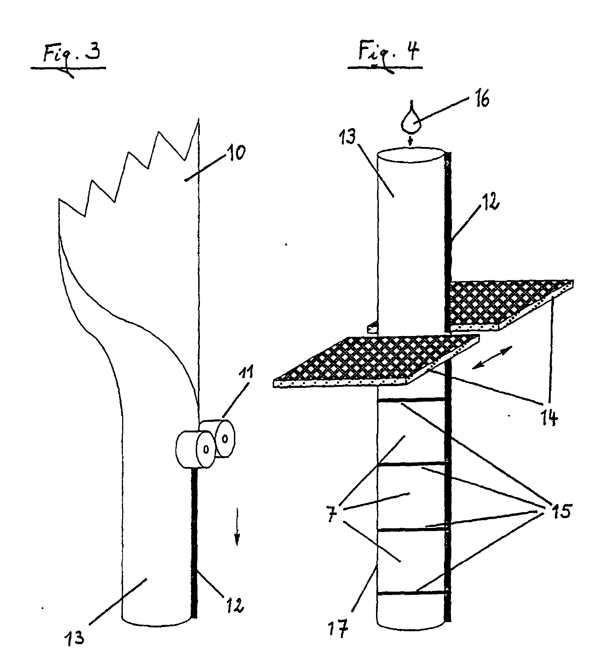

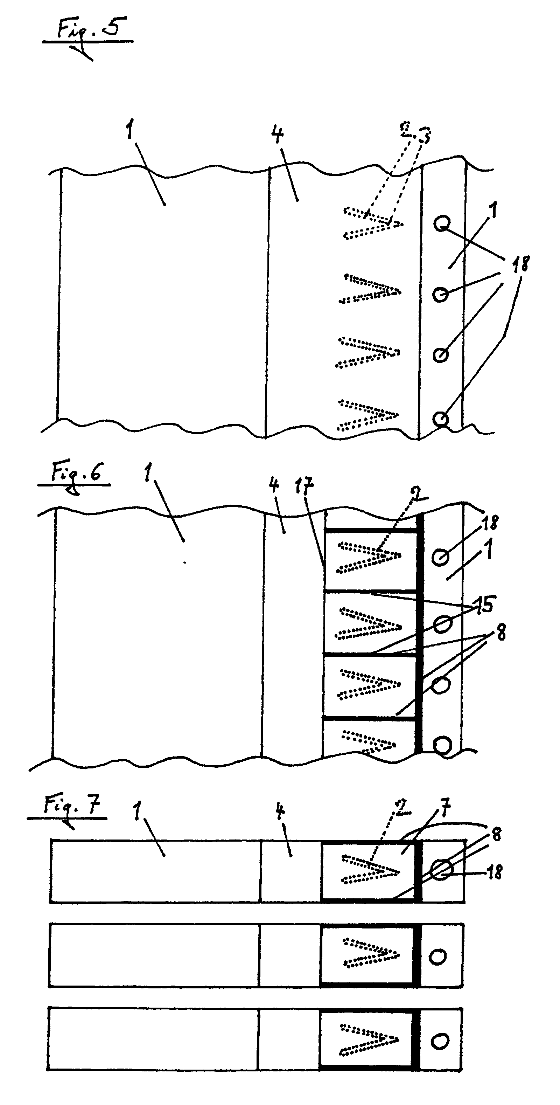

Für den Einsatz in erfindungsgemäßen analytischen Testelementen haben sich Blister als besonders vorteilhaft erwiesen, deren Herstellung in Fig. 3 und 4 mit der Darstellung charakteristischer Zwischenprodukte anschaulich gemacht wird. Das Herstellungsverfahren ist einfach durchzuführen und beinhaltet das in Fig. 3 dargestellte Verschweißen einer beispielsweise kunststoffkaschierten Metallfolie (10), beispielsweise einer kunststoffbeschichteten Alufolie an den Längsrändern der Folie zu einem kunststoffbeschichteten Metallschlauch (13). Aus einem kontinuierlichen, siegelfähigen Aluminiumband kann so im Durchlauf durch ein Paar geheizter Walzen, wie sie in Fig. 3 als Siegelrollen (11) dargestellt sind, ein Metallschlauch (13) gebildet werden. In Fig. 4 ist dargestellt, wie dann dieser Metallschlauch (13) durch ein Paar Heizbacken (14) im Taktverfahren quer zur Vorschubrichtung verschlossen wird. Wird der Schlauch von oben nach unten geführt, dann ist es möglich, oberhalb einer Querschweißnaht (15) Flüssigkeit (16) einzuspeisen und somit durch taktweises Querverschweißen flüssigkeitsgefüllte Blister (7) herzustellen, die zunächst als Blisterband (17) anfallen und die später vereinzelt werden können. Während es möglich ist, die Flüssigkeit (16) in einem zu dem Schließtakt der Heizbacken (14) zeitversetzten Takt in den Metallschlauch (13) einzufüllen, so daß in ein unten verschlossenes Schlauchstück eine gewisse Flüssigkeitsmenge eingefüllt und anschließend oberhalb des Flüssigkeitsniveaus mit Hilfe der Heizbacken (14) eine Querschweißnaht (15) gebildet wird, ist es auch möglich, nach einem ersten Verschließen des Metallschlauches (13) durch eine Querschweißnaht (15) eine größere Menge Flüssigkeit (16) in den nach unten geschlossenen Metallschlauch (13) einzufüllen und dann durch die Flüssigkeit (16) hindurch mit den Heizbacken (14) Querschweißnähte (15) zu bilden. Das letztere Verfahren hat den Vorteil, daß so praktisch keine Luft in die Blister (7) mit eingeschlossen wird.

Für ein bevorzugtes Herstellungsverfahren von bevorzugten erfindungsgemäßen analytischen Testelementen, wie sie in Fig. 1 dargestellt sind, sind wichtige Verfahrensstufen in Fig. 5 bis 7 dargestellt. Für die Herstellung solcher bevorzugter erfindungsgemäßer analytischer Testelemente wird ein Träger (1) in Form eines langen Bandes eingesetzt und mit nebeneinander angeordneten Einschnitten (2), die beim Biegen des Trägers (1) eine Spitze entstehen lassen, versehen. Über diese Einschnitte (2) wird das saugfähige Material (4) ebenfalls in Form eines langen Bandes auf den Träger (1) aufgebracht. Dieses Verfahrensstadium ist in Fig. 5 dargestellt.

Auf das saugfähige Material (4) wird ein Blisterband (17) so mit Schmelzkleber (8) befestigt, daß sich die mit Flüssigkeit gefüllten Blister (7) über den Einschnitten (2) befinden. Die Querschweißnähte (15) befinden sich jeweils zwischen zwei Einschnitten (2) angeordnet. Eine solche Anordnung wird durch die Positionierlöcher (18), die sich am Rande des als Band vorliegenden Trägers (1) angeordnet befinden, ermöglicht. Dieses Verfahrensstadium ist in Fig. 6 dargestellt.

Nachdem alle Verfahrensschritte erledigt sind, wird das vorliegende Band entlang der Querschweißnähte (15) des Blisterbandes (17) zerschnitten und so die einzelnen analytischen Testelemente gemäß der Erfindung bereitgestellt. Damit bei dieser Vereinzelung der erfindungsgemäßen analytischen Testelemente keine Flüssigkeit aus den Blistern (7) austritt, ist es natürlich erforderlich, daß die Querschweißnähte (15) entsprechend breit sind und auch der Schneidevorgang reproduzierbar und exakt so ausgeführt wird, daß die Querschweißnaht so zerschnitten wird, daß die Blister seitlich nicht geöffnet werden.

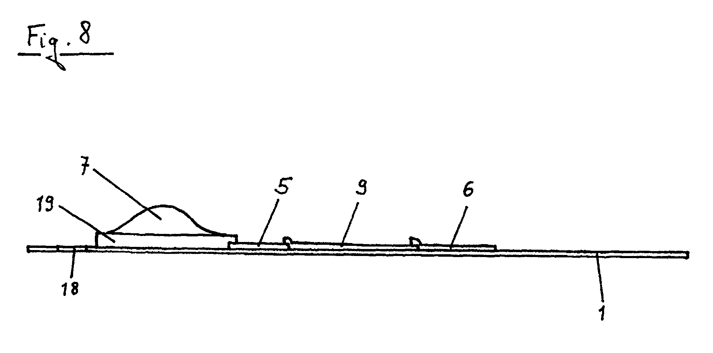

In Fig. 7 sind die vereinzelten analytischen Testelemente dargestellt.

Diese Testelemente sind insofern nicht nur einfach und damit preiswert herzustellen, sondern sie sind aufgrund ihrer Konstruktion einfach handhabbar für solche analytische Untersuchungen einsetzbar, die Flüssigkeit erfordern.

Die Erfindung wird durch die nachfolgenden Beispiele noch weiter erläutert.

### Beispiel 1: Herstellung eines Blisterbandes (17)

Eine 26 mm breite, 30 µm dicke Aluminiumfolie, die mit Haftvermittler und Polyethylen-Beschichtung von 15 g/m² ausgerüstet ist (Hersteller: Firma Alu-Singen, Singen, Deutschland) wird im Durchlaufverfahren thermisch zu einem Schlauch verschweißt. Die Nahtbreite beträgt 2,5 mm, die Rollentemperatur 180° C, die Geschwindigkeit 0,2m / min.

Unterhalb der Schweißstation ist eine Querschweißeinrichtung angeordnet. Die Querschweißstation arbeitet mit 2 Heizbacken, die eine Temperatur von 180° C aufweisen und eine Schweißzeit von 4 Sekunden. Der in der Längsschweißstation gebildete Schlauch wird bis zu einer Höhe von ca. 10 cm mit destilliertem Wasser aufgefüllt. In einem mittleren Abstand von 12 mm erfolgen Querverschweißungen. Die Breite der Nähte beträgt 4 mm.

### Beispiel 2: Herstellung des Trägers (1) in Form eines Bandes

Als Träger (1) dient eine 360 µm dicke Polyesterfolie (Typ Melinex, Hersteller Firma ICI, Dumfries, UK). Die Trägerfolie ist auf einer Breite von 40 mm mit Schmelzkleberpunkten von ca. 0,8 mm Durchmesser (Typ Dynapol, Hersteller Dynamit Nobel, Troisdorf, Deutschland) belegt. Auf einer Rollenstanzvorrichtung wird im Abstand von 12 mm ein Pilotloch sowie ein V-förmiger Einschnitt eingebracht. Auf einer Rollenschneidanlage wird die Folie auf 72 mm Breite beschnitten.

### Beispiel 3: Herstellung von erfindungsgemäßen analytischen Testelementen

Der Träger (1) aus Beispiel 2 wird zusammen mit einem 40 mm breiten Papier (Typ VLS 353, Hersteller: Firma Boehringer Mannheim GmbH, Mannheim, Deutschland) und dem Blisterband (17) nach Beispiel 1 quer zur Längsrichtung taktweise thermisch verklebt. Die Siegelabstände und Positionen werden über die Pilotlöcher gesteuert. Das entstehende Band wird aufgewickelt und in einem späteren Schritt auf einer lochgesteuerten Schneidemaschine in Streifen von 12 mm Breite geschnitten.

### Beispiel 4: Einfacher Funktionstest

auf eine glatte Oberfläche werden geringfügige Mengen von Kongorot in kristalliner Form aufgebracht. Mit dem Papierende eines nach Beispiel 3 hergestellten Testelementes wird die Oberfläche abgewischt. Es ist praktisch keine Verfärbung des Papieres mit bloßem Auge festzustellen.

Der Träger (1) des Testelementes wird anschließend im Bereich des V-förmigen Einschnitts geknickt und wieder gerade gebogen. Beim Knicken wird der Blister (7) perforiert, das Wasser tritt aus dem Blister (7) aus und wird durch Kapillarkräfte entlang des Papieres transportiert. In dem Bereich, in dem über die mit Kongorot kontaminierte Fläche gewischt worden ist, bildet sich sehr deutlich sichtbar eine rote Färbung.

Diese Beispiele zeigen die Möglichkeit, einen extrem einfachen Testaufbau zu realisieren. Das Prinzip ist auch für kompliziertere Nachweise brauchbar. So können durch Einbringung von reaktionsfähigen Systemen sowohl in die Flüssigkeit im Blister (7) als auch durch Eintränkungen in das Papier verschiedenste Nachweisreaktionen realisiert werden.

### Beispiel 5: Bestimmung von Cocain

### A. Herstellung der immunologischen Reagenzien

### a. Herstellung von Benzoylecgoninmaleimidoethylamid

2.4 g Benzoylecgoninhydrochlorid werden in 200 ml trockenem Acetonitril mit 1 g N-Hydroxysuccinimid und 1.8 g Dicyclohexylcarbodiimid versetzt und 3 Std. gerührt. Vom Niederschlag wird abfiltitriert, das Filtrat eingedampft, in Nitromethan aufgenommen und nochmals filtriert. Nach Abdampfen des Lösungsmittels wird mit Ether verrieben. Man erhält 1.13 g Benzoylecgoninsuccinimidylester. Dieses Produkt wird zusammen mit 0.47 g Maleimidoethylaminhydrochlorid (s. WO 90/15798) in 100 ml trockenem Acetonitril aufgenommen. Man gibt 1.1 g Triethylamin zu und rührt 12 Stunden bei Raumtemperatur. Das Reaktionsgemisch wird eingedampft, in 50 ml Essigester aufgenommen und drei Mal mit Natriumhydrogencarbonatlösung ausgeschüttelt. Die Essigesterphase wird eingedampft und das Produkt durch Aufnahme in 10 ml mit HCl gesättigtem Dioxan in das Hydrochlorid überführt. Man filtriert, wäscht mit Ether und erhält so 1 g Benzoylecgoninmaleimidoethylamidhydrochlorid.

### b. Herstellung des Cocain-Immunogens

300 mg Rinderserumalbumin werden in 25 ml 0.1 mol Kaliumphosphatpuffer pH 8.5 mit 106.6 mg S-Acetylthiopropionsäuresuccinimidylester gelöst in 5 ml Dioxan über 3 Stunden bei Raumtemperatur umgesetzt. Das modifizierte Rinderserumalbumin wird durch Gelchromatograhie über ACA 202 mit 0.1 mol Kaliumphosphatpuffer pH 8.5 von niedermolekularen Reaktionsprodukten abgetrennt. Man erhält eine Lösung von 310.5 mg Produkt in 57.5 ml Kaliumphosphatpuffer pH 8.5.
Eine Lösungsmenge, die 100 mg des modifizierten Rinderserumalbumin entspricht, wird mit 4.7 ml 1 molaren Hydroxylaminlösung umgesetzt. Danach gibt man 49.4 mg Benzoylecgoninmaleimidoethylamidhydrochlorid zu und läßt 12 Stunden bei 4°C reagieren. Das erhaltene Cocain-Immunogen wird durch Gelchromatograhie über ACA 202 mit 0.1 mol Kaliumphosphatpuffer pH 8.5 von niedermolekularen Reaktionsprodukten abgetrennt. Man erhält 95 mg Cocain-Immunogen gelöst in 0.1 molarem Kaliumphosphatpuffer pH 8.5.

### c. Gewinnung von Antikörpern gegen Cocain

10 Schafe wurden mit dem Cocain-Immunogen in komplettem Freundschen Adjuvans immunisiert. Die Dosis betrug jeweils 200 µg Immunogen pro Tier für die erste und jede Folgeimmunisierung. Die Immunisierungen erfolgten in monatlichen Abständen. Die erhaltenen Seren wurden in einem Mikrotiterplatten-Assay auf die Anwesenheit von Antikörpern gegen Cocain untersucht. Dazu wurden mit Streptavidin beschichtete Mikrotiterplatten mit Benzoylecgonin-[N'-biotinylaminocaproyl-(3,6-dioxa-8-aminooctyl)amid], hergestellt aus Benzoylecgoninsuccinimidylester und N-(Biotinylaminocaproyl)-1,8-diamino-3,6-dioxaoctan, inkubiert und gewaschen, danach mit den zu untersuchenden Seren inkubiert, wiederum gewaschen und zur Detektion mit einem Konjugat aus Peroxidase und einem Kaninchen-Anti-Schaf-Immunglobulin inkubiert, gewaschen und mit Substrat versetzt. Analog Beispiel 12 aus EPA 0 547 029 wurden die relativen Affinitäten zu Cocain bestimmt. Seren aus S 4987 mit einer guten Affinität gegenüber Cocain wurden für weitere Untersuchungen ausgewählt.

### d. Herstellung von Konjugaten aus Antikörpern gegen Cocain und alkalischer Phosphatase

Polyklonaler Antikörper gegen Cocain vom Schaf, DE-gereinigt (PAK<Cocain>S-IGG(DE)) wird aus delipidiertem Rohserum (Schaf) nach dem Fachmann bekannten Verfahren mittels Ammonsulfatfällung und DEAE-Sepharose-Chromatographie isoliert.

### Immunsorptive Reinigung von PAK<BZE>S-IGG(DE)

### Herstellung eines Cocain-Immunadsorbers

Das Cocain-Polyhapten aus Beispiel 5 e. (ohne Biotinylierungsschritt) wird an ein Glutardialdehyd-aktiviertes Affinitätsadsorbens (aktiviertes Spherosil; Boehringer Mannheim, Bestell-Nr. 665 525) entsprechend den Angaben des Herstellers gebunden.

### Immunsorption

(PAK<Cocain>S-IGG(DE)) wird gegen PBS/Azid (50 mmol/l Kaliumphosphat pH 7.5, 150 mmol/l NaCl, 0.1 % Na-Azid) dialysiert und anschließend innerhalb 2 Std. bei Raumtemperatur über eine geeignet dimensionierte Adsorbens-Säule (abhängig von Bindekapazität des Adsorbens und Titer des IGG(DE)) gegeben. Nach Auswaschen des nicht gebundenen Proteins mit PBS/Azid wird der gebundene Antikörper mit 1M Propionsäure bei Raumtemperatur eluiert. Das Eluat wird gegen 30 mM Natriumphosphat-Puffer pH 7.1 dialysiert.

### Herstellung von PAK<BZE>S-IGG(IS)-AP Konjugaten

### Aktivierung des IGG

Das immunsorptiv gereinigte IGG wird bei einer Konzentration von 10 mg Protein / ml in 30 mM Natriumphosphat-Puffer pH 7.1 mit 5 fach molarem Überschuß an Maleimido-hexanoyl-N-hydroxy-succinimidester (MHS) bei 25° C 1 Stunde inkubiert. Der Ansatz wird durch Zugabe von 100 fach molarem Überschuß an L-Lysin/HCl gegenüber MHS gestoppt und gegen 10 mM Kaliumphosphat, 50 mM NaCl, 10 mM MgCl₂, pH 6.1 dialysiert.

### Aktivierung von alkalischer Phosphatase (AP)

Die alkalische Phosphatase (EIA-Qualität, Boehringer Mannheim, Bestell-Nr. 567 744) wird bei einer Konzentration von 10 mg Protein / ml in 30 mM Triäthanolamin, 3M NaCl, 0.1 mM ZnCl₂, 1 mM MgCl₂, pH 7.0 mit 30 fach molarem Überschuß an Succinimidyl-acetylthiopropionat (SATP) bei 25°C 1 Stunde inkubiert. Der Ansatz wird durch Zugabe von L-Lysin/HCl ad 10 mM gestoppt und gegen 10 mM Kaliumphosphat, 50 mM NaCl, pH 7.5 dialysiert. Zur Deacetylierung der geschützten SH-Gruppe wird 1M Hydroxylamin-Lösung pH 7.5 ad 20 mM und 0.1M EDTA-Lösung ad 0.5 mM zugesetzt und 15 min bei 25°C inkubiert. Die aktivierte AP-Lösung wird sofort für die Kopplung eingesetzt.

### Kopplung

Die Lösungen der aktivierten AP sowie des aktivierten IGG werden äquimolar gemischt, der pH auf 6.8-7.0 und die AP-Konzentration mit bidest. Wasser auf 5 mg/ml eingestellt. Nach 3 Std. Reaktion bei 25°C wird der Ansatz durch sequentielle Zugabe von N-Ethylmaleimid (ad 5 mM, 30 min 25°C ) und 1M Hydroxylamin-Lösung pH 7.5 (ad 20 mM. 1 Std. 25°C) abgestoppt. Die Konjugat-Lösung wird gegen 50 mM Triäthanolamin/HCl, 150 mM NaCl, 1 mM MgCl₂, 0.1 mM ZnCl₂, pH 7.6 dialysiert und ad 10 mg/ml Rinderserumalbumin und 3 M NaCl aufgestockt.

### e. Herstellung eines biotinylierten Cocain-Polyhaptens

Kaninchen-IgG wird bei einer Konzentration von 25 mg/ml in Phospatpuffer pH 8 mit der 6-fach molaren Menge S-Acetylthiopropionsäuresuccinimidylester, gelöst in Dimethylsulfoxid, umgesetzt. Nach 1 Stunde bei 25 °C wird die Reaktion durch Zugabe einer Lösung von 1 mol/l Lysin gestoppt. Es folgt Dialyse gegen 0.1 mol/l Kaliumphosphatpuffer, pH 6 mit 1 mmol/l EDTA. Anschließend wird der pH auf 7.8 eingestellt und mit 1mol/l Hydroxylamin-Lösung, pH 7,5 ad 2o mmol/l 1 Stunde bei 25 °C inkubiert. Zur Kopplung wird ein 5fach molarer Überschuß Benzoylecgoninmaleimidoethylamidhydrochlorid in Dimethylsulfoxid gelöst und unter Rühren zu der Lösung des mit Sulfhydrylgruppen modifizierten Kaninchen-IgG gegeben. Nach Inkubation bei 25 °C für 2 Stunden wird die Reaktion gestoppt durch die successive Zugabe von 0.1 mol/l Cystein-Lösung ad 1mmol/l und 0.5 mol/l Jodacetamidlösung ad 5 mmol/l. Der Ansatz wird über Nacht gegen 0.1 mol/l Kaliumphosphatpuffer, pH 8.5 dialysiert und über Membranfiltration auf eine Proteinkonzentration von 10 mg/ml konzentriert. Danach wird das erhaltene Cocain-Polyhapten mit einem 8fach molarem Überschuß Biotinylcapronsäuresuccinimidylester, gelöst in Dimethylsulfoxid, biotinyliert.Der Ansatz wird gegen 20 mmol/l Natriumacetat, pH 4.3, dialysiert und über FPLC aufgereinigt.

### B. Aufbau eines analytischen Testelementes gemäß Fig. 8

### Blister tragende Zone (19)

Polyerstervlies der Firma Binzer, Hatzfeld, Bundesrepublik Deutschland. Es handelt sich um ein reines Polyestervlies, das mit 10% Kuralon verfestigt ist. Die Dicke beträgt 1,0 - 1,2 mm, die Saugkapazität 1800 ml/m².

### Probenauftragzone (5)

Ein Mischvlies aus 80 Teilen Polyester und 20 Teilen Zellwolle, verfestigt mit 20 Teilen Kuralon, in einer Dicke von 0,32 mm und mit einer Saugkapazität von 500 ml/m² wird mit folgender Lösung getränkt und anschließend getrocknet: 3,8 10⁻⁸ mol/l nach A. d. hergestelltes Konjugat aus Anti-Cocain-Antikörper und alkalischer Phosphatase, 3 mmol/l NaCl, 1 mmol/l MgCl₂, 0,1 mmol/l ZnCl₂, 30 mmol/l Triethylamin pH 7.6.

### Reagenzzone (9) als Abfangmatrix

Ein Vlies aus 100% Linters, verfestigt mit 2% Etadurin mit einer Dicke von 0.35 mm und einer Saugkapazität von 372 ml/m² wird mit folgender Lösung getränkt und anschließend getrocknet: 10 mmol/l Natriumphosphat pH 7.5, polymerisiertes Streptavidin 200 mg/l (Herstellung nach Beispiel 1d in EP-B-0 331 127).
Das vorgetränkte Vlies wird anschließend nochmals getränkt mit 10 mmol/l Natriumphosphat pH 7.5, 200 mg/l biotinyliertes Cocain-Polyhapten aus Beispiel A.e. und anschließend getrocknet.

### Nachweiszone (6)

Es wird ein Vlies aus 100% Linters, verfestigt mit 2% Etadurin mit einer Dicke von 0.35 mm und einer Saugkapazität von 372 ml/m² eingesetzt.

Die saugfähigen Matrices werden leicht überlappend auf eine Polyesterfolie (Typ Melinex, ICI, Dumfries, U.K) nach Beispiel 2 so geklebt, daß die Zone 19 über einem V-förmigen Einschnitt in der Polyesterfolie zu liegen kommt. Ein mit Pufferlösung (150 mmol/ 1 NaCl, 50 mmol / I Kaliumphosphatpuffer pH 7,2) gefülltes Blisterband, das analog Beispiel 1 hergestellt wird, wird auf dem Material der Zone 19 analog Beispiel 3 mit Schmelzkleber befestigt und anschließend werden Streifen von 12 mm Breite geschnitten, die die analytischen Testelemente gemäß Fig. 8 bilden.

### C. Messung

Cocain wird auf die Probenauftragzone (5) des analytischen Testelementes gemäß Fig. 8 gegeben. Danach wird der Träger (1) des Testelementes im Bereich des V-förmigen Einschnitts geknickt und wieder gerade gebogen. Bei Knicken wird der Blister (7) perforiert, die Pufferlösung tritt aus und wird von der Blister tragenden Zone (19) aufgenommen.

Die Flüssigkeit chromatographiert bis in die Nachweiszone (16). Aus dieser wird mit einem DIN 821 Locher ein rundes Teil ausgestanzt, mit der Vliesoberseite in ein Mikrotiterplatten-Well gelegt. In einem Lumineszenz-Mikrotiterplatten-Reader (Luminoscan) wird 100 µl Substratlösung (Lumiphos 530, Zubereitung von di-Natrium 3-(Methoxyspiro{1,2-dioxetan-3,2'-tricyclo[3.3.1.1.^{3,7}]decan}phenylphosphat, Boehringer Mannheim) zugegeben. Die Lumineszenz wird über den Zeitraum von 10 min gemessen.

Zur Bestimmung des Leerwertes wird ein Analysenelement verwendet, auf das kein Cocain gegeben worden ist.

### Meßwerte:

| beaufschlagte Menge Cocain [pg] | Lumineszenz frei. Einheiten / 60 sec.] |
|---|---|
| Leerwert | 60,46 |
| 1 | 76,86 |
| 10 | 106 |
| 100 | 134,4 |
| 1000 | 244,7 |

## Patentansprüche

1. Analytisches Testelement enthaltend auf einem Träger (1) saugfähiges Material (4) mit Probenauftrag- und Nachweiszone (5, 6) sowie einen flüssigkeitsgefüllten Blister (7), **dadurch gekennzeichnet, daß**
der Träger einen solchen Einschnitt (2) innerhalb der Trägerfläche besitzt, daß bei einem Biegen des Trägers dort eine Spitze (3) entsteht, mit der der Blister geöffnet werden kann, so daß Flüssigkeit austritt und in Kontakt mit dem saugfähigen Material gelangt.

2. Analytisches Testelement gemäß Anspruch 1, **dadurch gekennzeichnet, daß** sich der Blister in Kontakt mit dem saugfähigen Material befindet.

3. Analytisches Testelement gemäß einem der Ansprüche 1 oder 2, **dadurch gekennzeichnet, daß** Probenauftrag- und Nachweiszone bezüglich des saugfähigen Materials identisch sind.

4. Analytisches Testelement gemäß einem der Ansprüche 1 oder 2, **dadurch gekennzeichnet, daß** Probenauftrag- und Nachweiszone getrennte Bereiche sind.

5. Analytisches Testelement gemäß einem der Patentansprüche 1 oder 2, **dadurch gekennzeichnet, daß** Probenauftrag- und Nachweiszone aus unterschiedlichem Material bestehen.

6. Analytisches Testelement gemäß einem der Ansprüche 1-5, **dadurch gekennzeichnet, daß** der Einschnitt innerhalb der Trägerfläche V-förmig ist.

7. Analytisches Testelement gemäß einem der Ansprüche 1-6, **dadurch gekennzeichnet, daß** der Blister auf dem saugfähigen Material angeordnet ist.

8. Analytisches Testelement gemäß einem der Ansprüche 1-7, **dadurch gekennzeichnet, daß** der Blister direkt auf dem Träger befestigt ist.

9. Analytisches Testelement gemäß einem der Ansprüche 1-8, **dadurch gekennzeichnet, daß** der Blister auf dem Träger mit einer Folie (10) gehalten wird, die sich über den Blister spannt und vor und hinter dem Blister direkt auf dem Träger befestigt ist.

10. Analytisches Testelement gemäß einem der Ansprüche 1-9, **dadurch gekennzeichnet, daß** es sich so in einem stabilen Gehäuse befindet, daß der Träger so gebogen werden kann, daß eine Spitze entsteht, der mit der der Blister geöffnet werden kann und daß der Blister durch das Gehäuse so weit überdeckt ist, daß beim Biegen des Trägers ein Ausweichen des Blisters vor der Spitze verhindert wird.

11. Analytisches Testelement gemäß einem der Ansprüche 1-10, **dadurch gekennzeichnet, daß** das saugfähige Material außerhalb der Probenauftrag- und Nachweiszone mit einer flüssigkeitsundurchlässigen Folie so abgedeckt ist, daß es sich zwischen der Folie und dem Träger befindet.

12. Verfahren zur Herstellung eines analytischen Testelementes gemäß einem der Ansprüche 1-11, **dadurch gekennzeichnet, daß** auf einen Träger aus biegbarem aber dennoch steifem Material, der nebeneinander Einschnitte innerhalb der Trägerfläche aufweist, saugfähiges Material und ein kontinuierliches Band flüssigkeitsgefüllter Blister, die durch Querschweißnähte getrennt sind, so aufgebracht werden, daß ein Blister zwischen zwei Querschweißnähten jeweils über einen Einschnitt innerhalb der Trägerfläche zu liegen kommt und durch Schneiden entlang der Querschweißnähte im Blisterband einzelne analytische Testelemente entstehen.

13. Verfahren zur Bestimmung eines Analyts mittels eines analytischen Testelementes gemäß einem der Ansprüche 1-11, **dadurch gekennzeichnet, daß** Probe in Kontakt mit der Probenauftragzone gebracht, der Träger gebogen und mit der entstandenen Spitze der flüssigkeitsgefüllte Blister geöffnet wird, ausgetretene Flüssigkeit die Probe kontaktiert und entweder in der Probenauftragzone selbst oder in einer Nachweiszone, in die die Flüssigkeit transportiert wird, der Analyt bestimmt wird.

## Claims

1. Analytical test element containing on a support (1), absorbent material (4) with a sample application and detection zone (5, 6) as well as a blister (7) filled with liquid, **characterized in that**
the support has a cut (2) within the support surface such that a point (3) is formed when the support is bent that can be used to open the blister to allow liquid to escape and come into contact with the absorbent material.

2. Analytical test element as claimed in claim 1, **characterized in that** the blister is in contact with the absorbent material.

3. Analytical test element as claimed in one of the claims 1 or 2, **characterized in that** the absorbent material of the sample application and detection zone are identical.

4. Analytical test element as claimed in one of the claims 1 or 2, **characterized in that** the sample application and detection zone are separate areas.

5. Analytical test element as claimed in one of the claims 1 or 2, **characterized in that** the sample application and detection zone are composed of different materials.

6. Analytical test element as claimed in one of the claims 1 - 5, **characterized in that** the cut within the support surface is V-shaped.

7. Analytical test element as claimed in one of the claims 1 - 6, **characterized in that** the blister is located on the absorbent material.

8. Analytical test element as claimed in one of the claims 1 - 7, **characterized in that** the blister is attached directly to the support.

9. Analytical test element as claimed in one of the claims 1 - 8, **characterized in that** the blister is held on the support with a foil (10) which stretches over the blister and is attached directly to the support in front of and behind the blister.

10. Analytical test element as claimed in one of the claims 1 - 9, **characterized in that** it is located in a stable housing such that the support can be bent in such a manner that a point is formed that can be used to open the blister and the blister is covered by the housing to such an extent that it prevents the blister evading the tip when the support is bent.

11. Analytical test element as claimed in one of the claims 1 - 10, **characterized in that** the absorbent material outside the sample application and detection zone is covered with a liquid-impermeable foil in such a manner that it is disposed between the foil and support.

12. Process for the production of an analytical test element as claimed in one of the claims 1 - 11, wherein absorbent material and a continuous tape of blisters filled with liquid that are separated by transverse welding seams are applied to a support made of flexible but nevertheless stiff material which has cuts side by side within the support surface in such a manner that each blister between two transverse welding seams is disposed above a cut within the support surface and that individual analytical test elements are formed by cutting along the transverse welding seams in the blister tape.

13. Method for determining an analyte by an analytical test element as claimed in one of the claims 1 - 11, **characterized in that** the sample is contacted with the sample application zone, the support is bent and the blister containing liquid is opened by the point that is formed, the emerging liquid contacts the sample and the analyte is determined either in the sample application zone or in a detection zone into which the liquid is transported.

## Revendications

1. Elément d'essai analytique comprenant, sur un support (1), une matière absorbante (4) comprenant une zone d'application d'échantillon et une zone de décèlement (5, 6), ainsi qu'un blister (7) rempli de liquide, **caractérisé en ce que** le support possède une entaille (2) pratiquée dans la surface de support de telle sorte que, lorsqu'on plie le support on obtient à cet endroit une pointe (3) avec laquelle on peut ouvrir le blister, si bien que du liquide s'évacue et entre en contact avec la matière absorbante.

2. Elément d'essai analytique selon la revendication 1, **caractérisé en ce que** le blister est mis en contact avec la matière absorbante.

3. Elément d'essai analytique selon l'une quelconque des revendications 1 ou 2, **caractérisé en ce que** la zone d'application d'échantillon et la zone de décèlement sont identiques par rapport à la matière absorbante.

4. Elément d'essai analytique selon l'une quelconque des revendications 1 ou 2, **caractérisé en ce que** la zone d'application d'échantillon et la zone de décèlement représentent des zones séparées.

5. Elément d'essai analytique selon l'une quelconque des revendications 1 ou 2, **caractérisé en ce que** la zone d'application d'échantillon et la zone de décèlement sont constituées par une matière différente.

6. Elément d'essai analytique selon l'une quelconque des revendications 1 à 5, **caractérisé en ce que** l'entaille est pratiquée en V dans la surface de support.

7. Elément d'essai analytique selon l'une quelconque des revendications 1 à 6, **caractérisé en ce que** le blister est disposé sur la matière absorbante.

8. Elément d'essai analytique selon l'une quelconque des revendications 1 à 7, **caractérisé en ce que** le blister est fixé directement sur le support.

9. Elément d'essai analytique selon l'une quelconque des revendications 1 à 8, **caractérisé en ce que** le blister est maintenu sur le support à l'aide d'une feuille (10) qui s'étend par-dessus le blister et qui est fixée devant et derrière le blister directement sur le support.

10. Elément d'essai analytique selon l'une quelconque des revendications 1 à 9, **caractérisé en ce qu'**il se trouve dans un logement stable de telle sorte que le support peut être plié pour obtenir une pointe avec laquelle le blister peut être ouvert et **en ce que** le blister est recouvert par le logement à un point tel que lors du pliage du support, on empêche un affaissement du blister devant la pointe.

11. Elément d'essai analytique selon l'une quelconque des revendications 1 à 10, **caractérisé en ce que** la matière absorbante est recouverte, à l'extérieur de la zone d'application d'échantillon et de la zone de décèlement, d'une feuille imperméable au liquide qui est disposée entre la feuille et le support.

12. Procédé pour la fabrication d'un élément d'essai analytique selon l'une quelconque des revendications 1 à 11, **caractérisé en ce qu'**on applique, sur un support constitué d'une matière flexible, nonobstant rigide, qui présente des entailles juxtaposées pratiquées dans la surface de support, une matière absorbante et une bande continue de blisters remplis de liquide qui sont séparés par des cordons de soudure transversaux, de telle sorte qu'un blister entre deux cordons de soudure transversaux vient se disposer respectivement par-dessus une entaille pratiquée dans la surface de support et on obtient, par coupure le long des cordons de soudure transversaux, des éléments d'essais analytiques individuels dans la bande de blisters.

13. Procédé pour le décèlement d'un analyte à l'aide d'un élément d'essai analytique selon l'une quelconque des revendications 1 à 11, **caractérisé en ce qu'**on amène un échantillon en contact avec la zone d'application d'échantillon, on plie le support et on ouvre le blister rempli de liquide avec la pointe obtenue, le liquide qui s'évacue entre en contact avec l'échantillon et l'analyte est déterminé, soit dans la zone d'application d'échantillon elle-même, soit dans une zone de décèlement dans laquelle le liquide est transporté.
